# EUROPEAN PATENT APPLICATION

(11) **EP 1 972 285 A2**
(43) Date of publication of application: **24.09.2008**
(21) Application number: 08250454.9
(22) Date of filing: 07.02.2008
(51) Int. Cl.: A61B 17/02, G05G 5/06

(54) **Goose neck table post**

(30) Priority: 07.02.2007 US 888674 P
(71) Applicant: Codman & Shurtleff, Inc., Raynham, Massachusetts 02767-0350 (US)
(72) Inventor: Agbodoe, Victor, Stoughton, MA 02072 (US); Storz, Olaf, Tuttlingen 178532 (DE)
(74) Representative: Tunstall, Christopher Stephen

(57) **Abstract**

A table post for use during surgery includes a bar portion and an attachment portion. An annual mating saw connection is disposed between a distal end of the bar portion and a proximal end of the attachment portion. A handle is attached to the connection to selectively place the bar portion and the attachment portion in one of a fixed position with respect to each other and a moveable position. In the moveable position the attachment portion is free to rotate 360° with respect to the bar portion.

## Description

### FIELD OF THE INVENTION

The present invention relates to methods and devices for providing a goose neck table post.

### BACKGROUND OF THE INVENTION

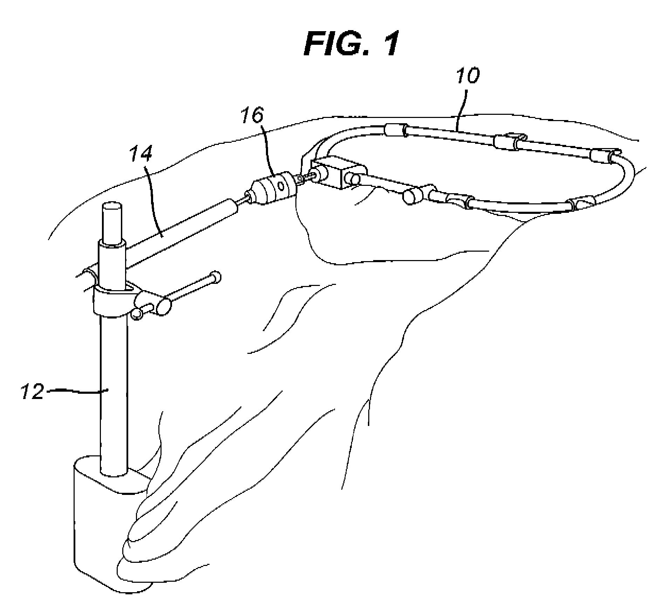

During surgery, retractors, such as those found in the BOOKWALTER^{™} retractor kit, which is commercially available from Codman & Shurtleff, Inc. of Raynham, MA, are often used to assist the surgeon and other operating room personnel to provide exposure to the surgical site for a broad range of surgical procedures. In surgical operations of the chest or abdomen, for example, it is often necessary to use a retraction apparatus to retain tissue away from the operative site. Typically, the retraction apparatus includes a housing member configured to lock onto a circumferential ring 10 located above the operative site (see Fig. 1). To maintain ring 10 in a fixed position, ring 10 can be connected to a support post 12 adjacent to the site by a horizontal bar 14 that has a flexible joint 16 (horizontal flex bar) to permit ring 10 to be oriented in various positions, as shown in Fig. 1. A horizontal extension arm may also be attached to the support post for supporting the circumferential ring. Within the housing member a retraction blade can usually be found for grabbing the tissue around the surgical incision. The housing member can also include a ratcheting mechanism and/or a tilting mechanism to draw the retraction blade away from the incision, thereby effecting the pulling away and/or lifting of the tissue around the incision to expose the desired surgical area. Examples of such retractor systems are disclosed in U.S. Pat. Nos. 4,254,763, 4,424,724 and 5,375,481, the disclosures of which are hereby incorporated by reference. During open surgical operations, such as, for example, open bariatric, ALIF procedures, hepatic resections, transplant procedures, abdominal aortic aneurysms, hernia repair, appendectomy and others, many different instruments are used, such as, for example, a retractor blade with ring attachment systems are used. In some of these operations, the patient can be overweight. Thus, there is a need for a post that provides an integral turn while permitting the distal end to rotate 360 degrees.

Thus, despite these retraction systems, there continues to be a need for a retraction system that can accommodate an overweight patient while permitting the distal end of the post to rotate 360 degrees.

### SUMMARY OF THE INVENTION

### BRIEF DESCRIPTION OF THE DRAWINGS

The invention will be more fully understood from the following detailed description taken in conjunction with the accompanying drawings, in which:

FIG. 1 is a plan view of a conventional retractor system;

FIG. 2 is a plan view of goose neck table post in accordance with the present invention; and

FIG. 3 is a side view of the goose neck table post with parts broken away.

### DETAILED DESCRIPTION OF THE INVENTION

Certain exemplary embodiments will now be described to provide an overall understanding of the principles of the structure, function, manufacture, and use of the devices and methods disclosed herein. One or more examples of these embodiments are illustrated in the accompanying drawings. Those skilled in the art will understand that the devices and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments. Such modifications and variations are intended to be included within the scope of the present invention.

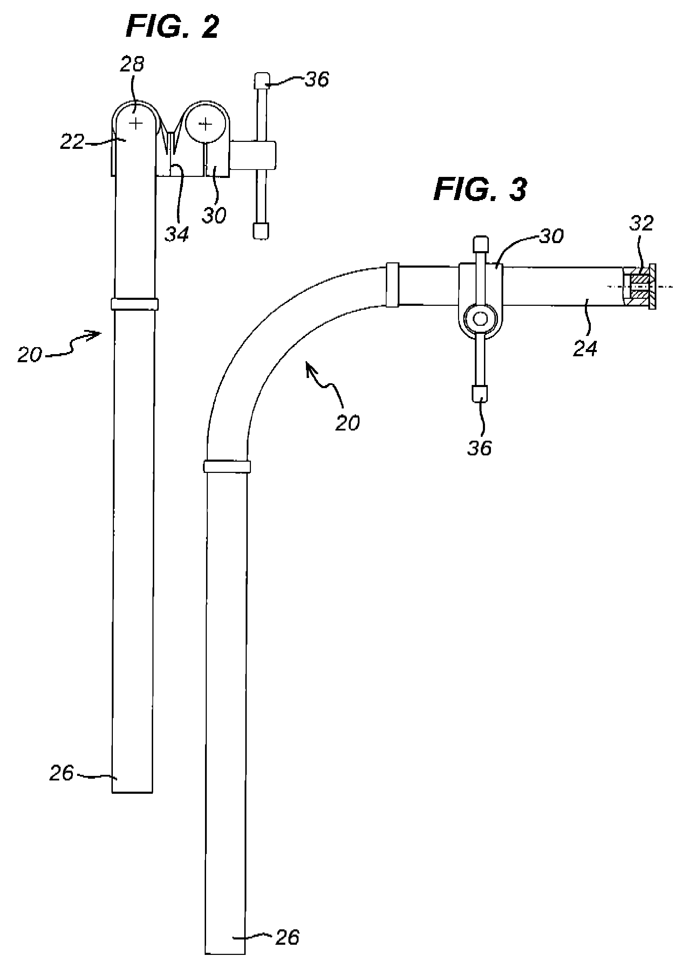

The present invention provides methods and devices for holding a retractor ring. A goose neck post holder 20 is illustrated in Figs. 2 and 3. Holder 20 includes a bar portion 22 and an attachment portion 24. Bar portion 22 has a proximal end 26 and a distal end 28. Bar portion 22 includes a linear section and a curved section. The curved section is adjacent to the distal end of the bar portion, and extends for an arc angle of approximately 90°. Attachment portion 24 includes a proximal end 30 and a distal end 32. Proximal end 26 can connect, for example, to a table post in a manner known to those skilled in the art. Distal end 28 of bar portion 22 is selectively fixedly connected to attachment portion 24 by an annual mating serrated saw-like connection 34, which may also be referred to as a starburst connection. Connection 34 is selectively loosened and tightened by rotating handle 36 in either a clockwise or counterclockwise direction. In the loosened position, attachment portion 24 is free to rotate 360 degrees with respect to bar portion 22. In the tightened position, however, attachment portion 24 is fixedly connected to bar portion 22.

Distal end 32 of attachment portion 24 may connect to a horizontal bar, a post coupling or a ring holder in a manner known to those skilled in the art. Thus, the goose neck post holder 20 in accordance with the present invention provides a retraction system that can accommodate an overweight patient while permitting the distal end of the post to rotate 360 degrees.

One skilled in the art will appreciate further features and advantages of the invention based on the above-described embodiments. Accordingly, the invention is not to be limited by what has been particularly shown and described, except as indicated by the appended claims. All publications and references cited herein are expressly incorporated herein by reference in their entirety.

## Claims

1. A table post for use during surgery comprising:
a bar portion having a proximal end configured to be connected to a table post and a distal end;
an attachment portion having a proximal end and a distal end, said distal end being configured to be connected to one of a horizontal bar, a post coupling and a ring holder;
an annual mating saw connection being disposed between said distal end of said bar portion and said proximal end of said attachment portion;
a handle being attached to said connection to selectively place said bar portion and said attachment portion in one of a fixed position with respect to each other and a moveable position, in said moveable position said attachment portion is free to rotate 360° with respect to said bar portion.

2. The table post according to claim 1, wherein said handle is rotatable.

3. The table post according to claim 1, wherein said bar portion includes a linear section and a curved section.

4. The table post according to claim 3, wherein said curved section is adjacent to said distal end of said bar portion.

5. The table post according to claim 4, wherein said curved section has an arc angle of about 90°.
